# EUROPEAN PATENT APPLICATION

(11) **EP 2 733 948 A1**
(43) Date of publication of application: **21.05.2014**
(21) Application number: 13164055.9
(22) Date of filing: 17.04.2013
(51) Int. Cl.: H04N 13/04

(54) **Method and apparatus for outputting image, system for providing image using the same**

(30) Priority: 20.11.2012 KR 20120131955
(71) Applicant: Samsung Medison Co., Ltd., Gangwon-do 250-870 (KR)
(72) Inventor: Lee, Jun-kyo, Gangwon-do (KR); Kim, Han-jun, Gangwon-do (KR); Kim, Sung-yun, Gangwon-do (KR); Yoo, Jun-sang, Gangwon-do (KR)
(74) Representative: Lorenz, Markus

(57) **Abstract**

An image output apparatus including a filtering unit configured to filter a first superimposed image including a plurality of image data regarding a target object and to output a second superimposed image including the plurality of image data at a predetermined ratio, and a controller configured to control the filtering unit to vary the ratio based on an external input.

## Description

### CROSS-REFERENCE TO RELATED PATENT APPLICATION

This application claims the benefit of Korean Patent Application No. 10-2012-0131955, filed on November 20, 2012, in the Korean Intellectual Property Office, the disclosure of which is incorporated herein in its entirety by reference.

### BACKGROUND OF THE INVENTION

### 1. Field of the Invention

The present invention relates to a method and apparatus for outputting images and a method and system for providing images. More particularly, the present invention relates to a method and system for providing a superimposed image including a plurality of medical image data and a method and apparatus for outputting images used for the system and method.

### 2. Description of the Related Art

An ultrasonic diagnosis apparatus using ultrasonic waves is being widely used in various medical fields to obtain information regarding the inside of a living body test target due to its non-invasive and non-destructive diagnostic characteristics. Since the ultrasonic diagnosis apparatus may provide images of internal tissues of the human body to doctors in real-time without the need for observation techniques that involve invading human tissues, such as surgery, the use of ultrasonic diagnosis apparatuses is important in the medical field. Recently, a highly efficient ultrasonic diagnosis apparatus has been employed to generate 2-dimensional or 3-dimensional diagnostic images of internal shapes of a target object.

In recent years, the rapid progress of image display techniques has led to various attempts at realizing stereoscopic images capable of mimicking human sight, which, among all of the human senses, involves the largest amount of information acquisition, and thus, there has been rapid technical advancement.

Stereoscopic images may be embodied by the principle that by varying angles of images recognized by both eyes of a user, when viewing images displayed on a plane surface, the user experiences a stereoscopic effect as if he or she is viewing an actual object. Methods of embodying the stereoscopic images may include glasses-type methods and non-glasses-type methods. Methods of varying left and right image data displayed using glasses may include an anaglyph method using a red lens and a blue lens, a polarization method using a polarization filter, and a shutter method using shutter glasses. Also, methods of varying image data recognized by left and right eyes according to a user's sight without glasses may include a parallax barrier method and a lenticular lens method.

### SUMMARY OF THE INVENTION

The present invention provides a method and apparatus for outputting images and a method and system for providing images, which may allow each of a plurality of users to selectively view an interesting image on one screen configured to output a superimposed image including a plurality of image data.

Also, the present invention provides a method and apparatus for outputting images and a method and system for providing images, which may allow a user to easily control a ratio between a plurality of image data included in a superimposed image.

According to an aspect of the present invention, there is provided an image output apparatus including: a filtering unit configured to filter a first superimposed image including a plurality of image data regarding a target object and to output a second superimposed image including the plurality of image data at a predetermined ratio, and a controller configured to control the filtering unit to vary the ratio based on an external input. The controller may receive the external input using at least one of a wheel, a button, a wireless remote controller, a speech recognition sensor, and a gesture recognition sensor.

The apparatus may further include a position information acquisition unit configured to obtain user position information. The controller may control the filtering unit based on the obtained user position information.

The controller may control the filtering unit such that the second superimposed image consists of at least one image data from among the plurality of image data.

The filtering unit may filter the first superimposed image using at least one of an anaglyph method, a shutter method, a polarization method, a parallax barrier method, and a lenticular lens method.

The filtering unit using the anaglyph method may control colors filtered through the filtering unit and filter the first superimposed image, and the filtering unit using the shutter method may control opening and closing of a shutter included in the filtering unit and filter the first superimposed image. Also, the filtering unit using the polarization method may control a polarization angle of a polarization filter included in the filtering unit and filter the first superimposed image, and the filtering unit using the parallax barrier method may control a visual field cut off by a parallax barrier included in the filtering unit and filter the first superimposed image. Furthermore, the filtering unit using the lenticular lens method may control an angle refracted by a lenticular lens included in the filtering unit and filter the first superimposed image.

According to another aspect of the present invention, there is provided a method of outputting images, including: filtering, by a filtering unit, a first superimposed image including a plurality of image data regarding a target object and outputting a second superimposed image including the plurality of image data at a predetermined ratio, and controlling the filtering unit to vary the ratio based on an external output.

According to another aspect of the present invention, there is provided an image providing system including: an image generator configured to generate a first superimposed image including a plurality of image data regarding a target object, a display unit being configured to display the first superimposed image, and an image output unit including a filtering unit and a controller, the filtering unit configured to filter the first superimposed image and to output a second superimposed image including the plurality of image data at a predetermined ratio, the controller being configured to control the filtering unit to vary the ratio. The controller may control the filtering unit to vary the ratio based on an external input.

The image generator included in the image providing system may generate the first superimposed image including at least two image data obtained using different medical imaging methods.

The image generator may generate the first superimposed image including a first image and a second image. The first image may show the target object, and the second image may show additional information regarding the target object. For example, the first image may include at least one of an ultrasonic image, a magnetic resonance (MR) image, a computed tomography (CT) image, and a positron emission tomography (PET) image of the target object. The additional information included in the second image may include at least one of information regarding numerical values of the target object, annotation, a marker, and color Doppler data.

According to another aspect of the present invention, there is provided a method of providing images, including: generating a first superimposed image including a plurality of image data regarding a target object, displaying the first superimposed image, filtering, by the filtering unit, the first superimposed image and outputting a second superimposed image including the plurality of image data at a predetermined ratio, and controlling the filtering unit to vary the ratio based on an external input.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of an image output apparatus according to an embodiment of the present invention;
FIG. 2 is a flowchart illustrating a method of outputting images, according to an embodiment of the present invention;
FIGS. 3A to 3C are diagrams for explaining an image output apparatus configured to output superimposed images including a plurality of image data having a varied ratio there between, according to an embodiment of the present invention;
FIG. 4 is a diagram for explaining an image output apparatus configured to selectively provide a plurality of data included in a superimposed image using a parallax barrier;
FIG. 5 illustrates examples of a first superimposed image including a plurality of image data being filtered by an image output apparatus, according to an embodiment of the present invention;
FIG. 6 is a diagram for explaining an image output apparatus configured to receive an external input using a wheel, according to an embodiment of the present invention;
FIG. 7 is a diagram for explaining an image output apparatus capable of outputting different image data to a plurality of users, according to an embodiment of the present invention;
FIG. 8 is a block diagram of an image providing system according to an embodiment of the present invention; and
FIG. 9 is a flowchart illustrating a method of providing images, according to an embodiment of the present invention.

### DETAILED DESCRIPTION OF THE INVENTION

As used herein, expressions such as "at least one of," when preceding a list of elements, modify the entire list of elements and do not modify the individual elements of the list.

The present invention is described more fully hereinafter with reference to the accompanying drawings, in which embodiments of the invention are shown.

While terms used in the present invention are selected, wherever possible, from terms currently in widespread use, the terms may vary according to an operator's intention, judicial precedents, or the emergence of new technology. In exceptional cases, terms arbitrarily selected by the applicants may be used. In these cases, the meanings of the terms should be understood in consideration not of simple names of the terms but of meanings described or used in the detailed description of the present invention.

It will be further understood that the terms "comprises," "comprising," "includes," and/or "including," when used herein, specify the presence of stated features, items, steps, operations, elements, and/or components, but do not preclude the presence or addition of one or more other features, items, steps, operations, elements, components, and/or groups thereof. Also, the term "unit" refers to a unit configured to process at least one function or operation and may be embodied by hardware, software, or a combination of hardware and software.

In this specification, the term "object" may be a creature or a lifeless thing whose image is to be captured. Also, the term "object" may refer to a portion of the human body and include internal organs, such as the liver, the heart, the womb, the brain, the breast, and the abdomen, or fetuses. In addition, the object may include a cross-section of a portion of the human body. In this specification, the term "user" may refer to a medical expert, such as a doctor, a nurse, a clinical pathologist, and a medical image expert, or a patient or an ordinary person that desires or intends to obtain medical images, but is not limited thereto.

Furthermore, images may include medical images for diagnosing diseases, such as ultrasonic images, magnetic resonance (MR) images, computed tomography (CT) images, and positron emission tomography (PET) images, but the present invention is not limited thereto.

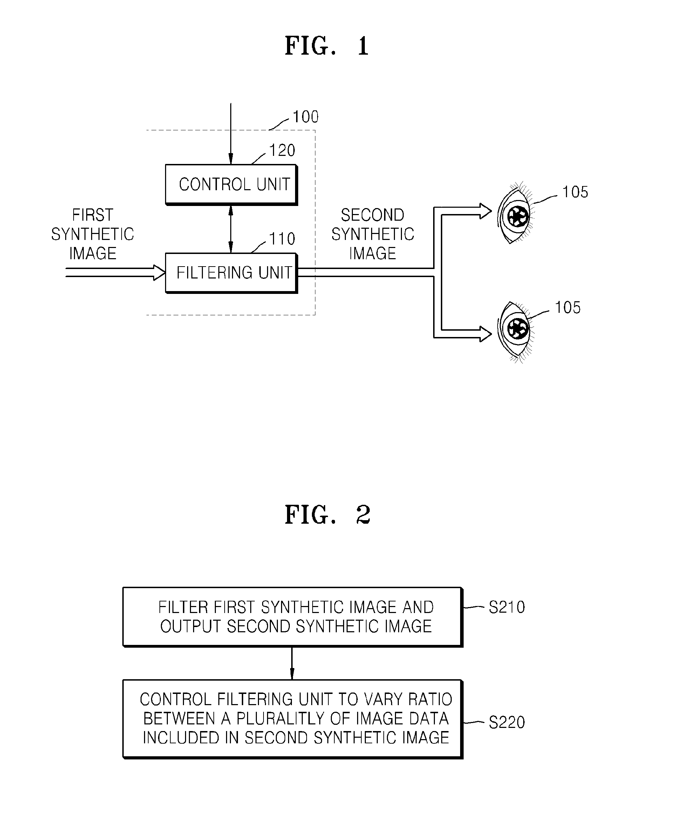

FIG. 1 is a block diagram of an image output apparatus 100 according to an embodiment of the present invention.

The image output apparatus 100 according to the present embodiment may include a filtering unit 110 and a controller 120. However, the image output apparatus 100 may be embodied by a larger or smaller number of components than those shown. Hereinafter, the above-described components will be described in detail.

The filtering unit 110 may filter a first superimposed image including a plurality of image data regarding a target object and output a second superimposed image including the plurality of image data at a predetermined ratio relative to each other. That is, while the first superimposed image is being filtered through the filtering unit 110, a ratio between the plurality of image data that is included in the first superimposed image may be varied. The second superimposed image output by the filtering unit 110 may be provided to a user 105.

The controller 120 may control the filtering unit 110 based on an external input. In this case, the controller 120 may control the filtering unit 110 to vary a ratio in which the plurality of image data is included in the second superimposed image output by the filtering unit 110. Also, the controller 120 may control the overall operation of the image output apparatus 100. For example, the controller 120 may execute programs stored in a memory (not shown) and control the filtering unit 110. Furthermore, the controller 120 may include a receiver (not shown) configured to receive an external input. The controller 120 may control the filtering unit 110 based on the external input. For instance, the image output apparatus 100 according to the present embodiment may be in the form of glasses. In this case, the filtering unit 110 may be included in lenses of the glasses.

FIG. 2 is a flowchart illustrating a method of outputting images according to an embodiment of the present invention.

Referring to FIG. 2, the method of outputting images according to the present embodiment may include operations processed by the image output apparatus 100 shown in FIG. 1. Accordingly, even if omitted below, the above description of the image output apparatus 100 shown in FIG. 1 may be applied to the method of outputting images shown in FIG. 2.

In operation S210, the image output apparatus 100 may filter a first superimposed image including a plurality of image data regarding a target object and output a second superimposed image including the plurality of image data at a predetermined ratio. Operation S21 0 may be performed by the filtering unit 110.

In this case, "the first superimposed image including the plurality of image data" may be a "fusion image". The fusion image refers to an image obtained by overlaying or merging a plurality of images to provide additional information. The fusion image may be generated from a plurality of images using the same image method or from a plurality of images using several image methods. The image methods may include an ultrasonic image method, a magnetic resonance (MR) image method, a computed tomography (CT) image method, and a positron emission tomography (PET) image method.

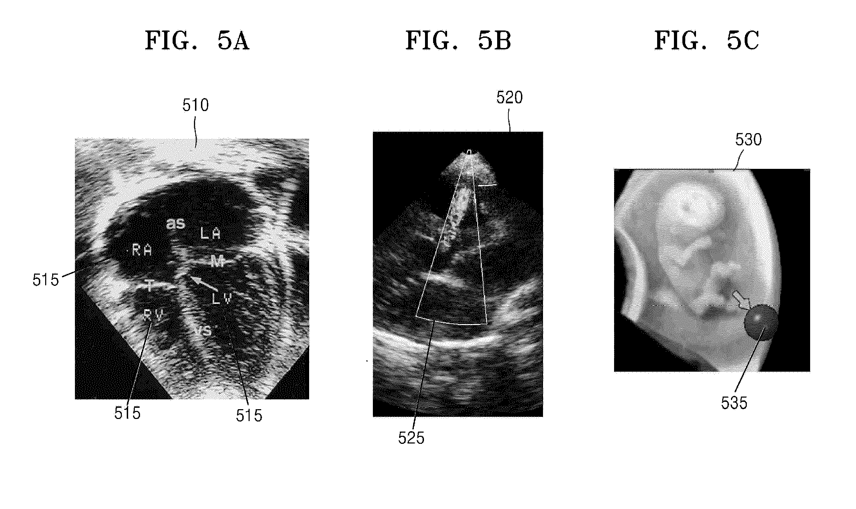

Furthermore, "the first superimposed image including the plurality of image data" may refer to an image obtained by indicating additional information regarding a target object on a medical image of the target object. For example, the image of the target object may be an ultrasonic image of the target object, an MR image of the target object, a CT image of the target object, or a PET image of the target object. Also, the additional information regarding the target object may be, for example, information regarding numerical values of the target object, an annotation, a marker, or color Doppler data. The first superimposed image including the additional information regarding the target object will be described in further detail later with reference to FIG. 5.

Meanwhile, the filtering unit 110 included in the image output apparatus 100 may filter a first superimposed image using an anaglyph method so that the image output apparatus 100 can output the second superimposed image including a plurality of image data in the predetermined ratio in operation S210. The filtering unit 110 using the anaglyph method, which may include a color filter, may control colors filtered through the filtering unit 110 and selectively filter image data. For example, the first superimposed image filtered by the filtering unit 110 may be an image obtained by overlaying two images in different colors.

The filtering unit 110 included in the image output apparatus 100 may filter the first superimposed image using a shutter method. The filtering unit 110 using the shutter method may control opening and closing of a shutter and control an image filtered through the filtering unit 110 so that image data can be selectively filtered. For example, the first superimposed image filtered by the filtering unit 110 may be an image output by alternating two images.

The filtering unit 110 included in the image output apparatus 100 may filter the first superimposed image using a polarization method. The filtering unit 110 using the polarization method may selectively filter the image data by controlling a polarization angle at which image data is filtered through the filtering unit 110 using a polarization filter. For example, the first superimposed image filtered by the filtering unit 110 may be an image obtained by overlaying two images having different polarization angles.

The filtering unit 110 included in the image output apparatus 100 may filter the first superimposed image using a parallax barrier method. The first superimposed image filtered by the filtering unit 110 may be displayed such that a plurality of image data are alternately disposed along a scan line. A parallax barrier may include a plurality of slits and is used to partially cut off a user's visual field of the displayed first superimposed image. Accordingly, the image output apparatus 100 using the parallax barrier method may control image data that is cut off by a barrier included in the parallax barrier and image data that is provided to a user through the slits, and thus, the image data can be selectively filtered.

The filtering unit 110 included in the image output apparatus 100 may filter the first superimposed image using a lenticular lens method. The first superimposed image filtered by the filtering unit 110 may be displayed such that the plurality of image data are alternately disposed along a scan line. Accordingly, the image output apparatus 100 using the lenticular lens method may control a refraction angle of image data using a lenticular lens and refract the image data such that only selected image data is provided to a user.

In operation S220, the image output apparatus 100 may control the filtering unit 110 based on an external output. Operation S220 may be performed by the controller 120. In this case, the external input may be received by the controller 120 using a physical method, such as an operation of rotating a wheel, which may be performed by a user. Also, the external input may be received by the controller 120 using an electrical method, such as an operation of inputting an electric signal by pushing a button, an operation of recognizing a user's speech, an operation of recognizing a user's gesture, and an operation of receiving a signal transmitted by a remote controller. The image output apparatus 100 may control the filtering unit 110 based on the external input and control a ratio between a plurality of image data included in a superimposed image so that the image data can be selectively provided to the user. Accordingly, in the image output apparatus 100 according to the present embodiment, a plurality of users may selectively view interesting images on only one screen without interfering with each other. Also, in the image output apparatus 100 according to the present embodiment, a user may remove image data including noise or artifacts from a plurality of image data and view a superimposed image so that the remaining image data can be precisely determined without the influence of the noise or artifacts.

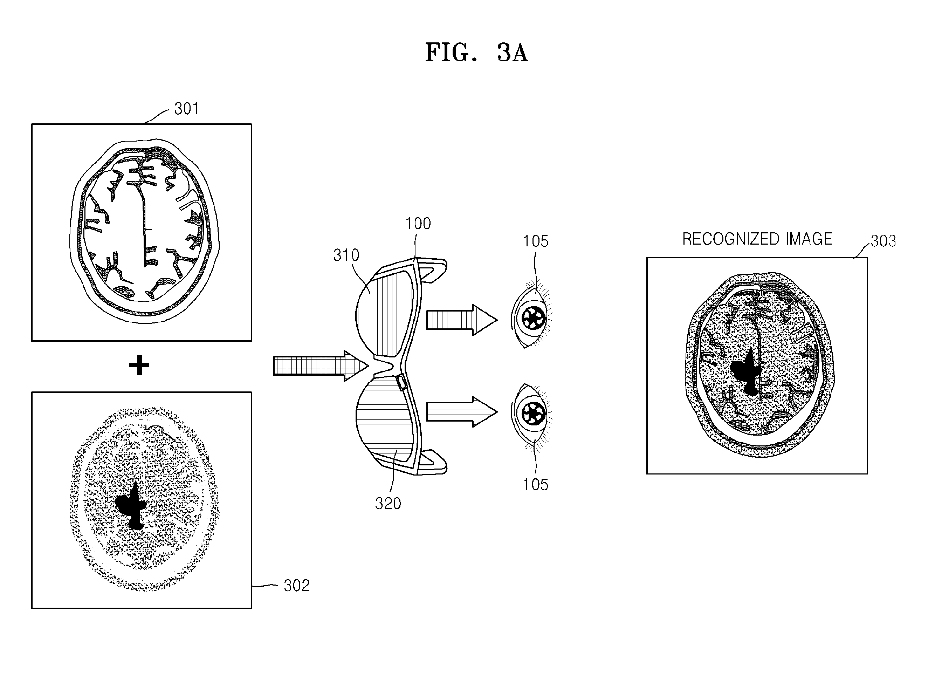

Furthermore, the image output apparatus 100 according to the present embodiment may obtain user position information and control a ratio between the plurality of image data included in the second superimposed image output by the filtering unit 110, based on the obtained position information. For example, the user position information may include a distance between a predetermined display device on which the first superimposed image is displayed and a user, or an angle of the user relative to the display device. Accordingly, in the image output apparatus 100 according to the present embodiment, a plurality of users located in different positions may receive different images from one display device. Also, a user may select desired image data according to a position in which the user stands. FIGS. 3A to 3C are diagrams for explaining an image output apparatus 100 configured to output a second superimposed image including a plurality of image data having a varied ratio there between, according to an embodiment of the present invention.

As shown in FIGS. 3A to 3C, the image output apparatus 100 according to the present embodiment may be provided in the form of glasses to a user 105. In an example, the image output apparatus 100 shown in FIGS. 3A to 3C may control a filtering unit 110 including a polarization filter and vary a ratio between a plurality of image data included in an output image. In FIGS. 3A to 3C, a first superimposed image filtered by the filtering unit 110 may be an image obtained by overlaying two images having different polarization angles. The first superimposed image may be an image obtained by overlaying an MR image 301 and a PET image 302, each of which shows a cross-section of the human brain. For example, the MR image 301 and the PET image 302 may be input in alternating scan lines of a display device and pass through the polarization filter adhered on a front surface of a liquid crystal (LC) panel having different polarization characteristics and generate the first superimposed image. In FIGS. 3A to 3C, the first superimposed image may include a horizontally polarized MR image 301 and a vertically polarized PET image 302.

FIG. 3A illustrates an image output apparatus 100 configured to output a second superimposed image including an MR image 301 and a PET image 302 at a ratio of 1: 1. In this case, the controller 120 may control a polarization angle of a polarization filter such that the right-eye filtering unit 310 of the image output apparatus 100 filters only a horizontally polarized light beam, and may control the polarization angle of the polarization filter such that the left-eye filtering unit 320 of the image output apparatus 100 filters only a vertically polarized light beam. When the first superimposed image is filtered through the filtering units 310 and 320, only the MR image 301 may be output to the user's right eye, and only the PET image 302 may be output to the user's left eye. As a result, the second superimposed image recognized by the user may be the same as the image 303 including the MR image 301 and the PET image 302 at a ratio of about 1: 1.

In another example, the controller 120 may control the filtering unit 110 not to have polarization characteristics so that the image output apparatus 100 can output the second superimposed image including the MR image 301 and the PET image 302 at a ratio of about 1: 1. That is, the first superimposed image into which the horizontally polarized MR image 301 and the vertically polarized PET image 302 are merged may be filtered through the filtering unit 110 and directly output to both eyes of the user, so that he or she can recognize an image 303 including the MR image 301 and the PET image 302 at a ratio of about 1: 1.

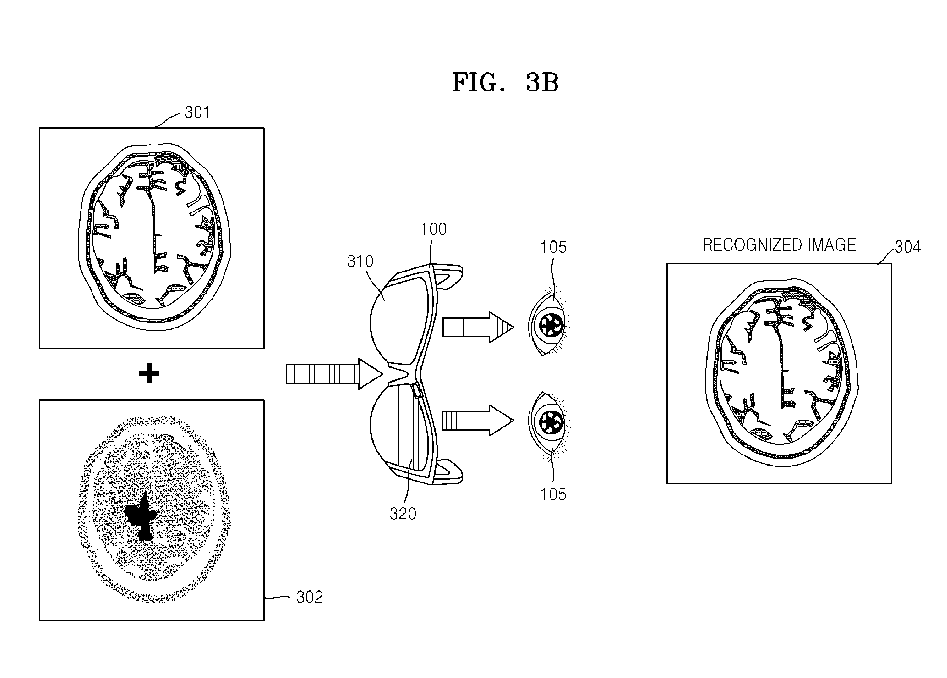

FIG. 3B illustrates an image output apparatus 100 configured to output a second superimposed image including an MR image 301 and a PET image 302 at a ratio of about 1:0. In this case, the controller 120 may control a polarization angle of the polarization filter such that the left- and right-eye filtering units 310 and 320 of the image output apparatus 100 filter only horizontally polarized light beams. When the first superimposed image is filtered through the filtering units 310 and 320, only an MR image 301 may be output to both eyes of the user. Accordingly, the second superimposed image recognized by the user may be the same as an image 304 including the MR image 301 and the PET image 302 at a ratio of about 1: 0.

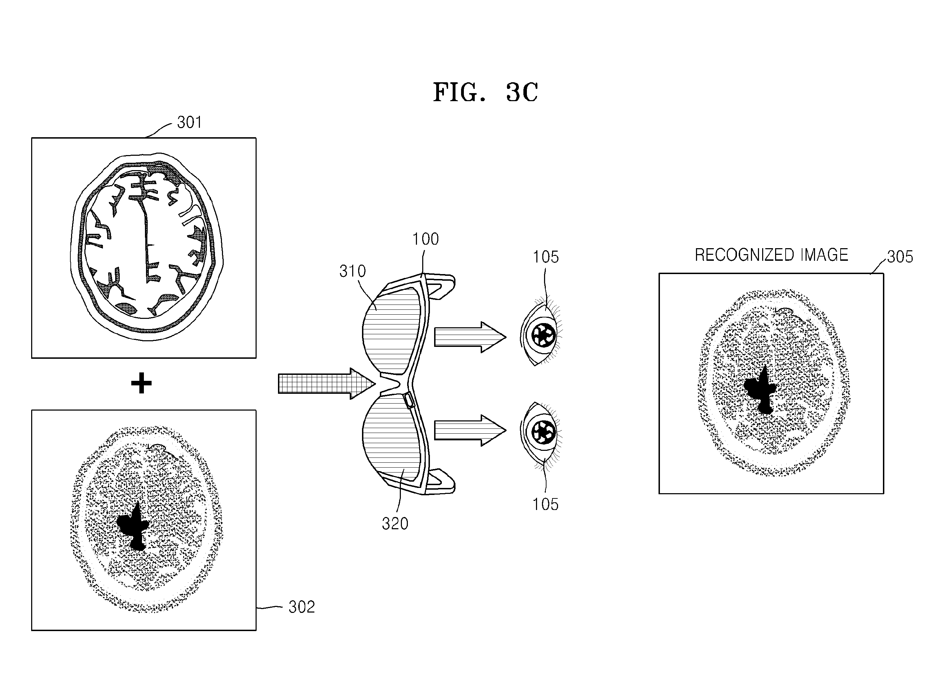

FIG. 3C illustrates an image output apparatus 100 configured to output a second superimposed image including an MR image 301 and a PET image 302 at a ratio of about 0:1. In this case, the controller 120 may control a polarization angle of a polarization filter such that left- and right-eye filtering units 310 and 320 of the image output apparatus 100 filter only vertically polarized light beams. When a first superimposed image is filtered through the filtering units 310 and 320, only the PET image 302 may be input to both eyes of the user. As a result, the second superimposed image recognized by the user may be the same as an image 305 including the MR image 301 and the PET image 302 at a ratio of about 0:1.

As shown in FIGS. 3A to 3C, a controller 120 included in the image output apparatus 100 according to the present embodiment may control the filtering unit 110 and selectively output a plurality of image data. Also, the filtering unit 110 may include a left-eye filtering unit 310 and a right-eye filtering unit 320. The controller 120 may additionally control the left-eye filtering unit 310 and the right-eye filtering unit 320 to output different images to both eyes of the user.

Meanwhile, image output methods for varying image data recognized by a user according to a parallax effect may include a parallax barrier method and a lenticular lens method. By using a lenticular lens or a parallax barrier, a plurality of image data or partial data included in a superimposed image being displayed may be dividedly output to left and right eyes or simultaneously output to both eyes. In an embodiment of the present invention, the image output apparatus 100 may control the parallax barrier or the lenticular lens based on an external input and selectively output image data. For example, the image output apparatus 100 may obtain user position information and control the parallax barrier or the lenticular lens based on a user's position to selectively output image data.

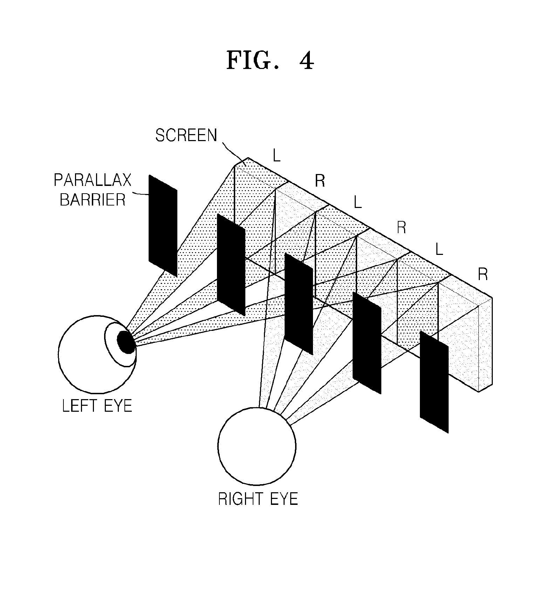

FIG. 4 is a diagram for explaining an image output apparatus configured to selectively provide a plurality of data included in a superimposed image using a parallax barrier.

As shown in FIG. 4, a superimposed image including L image data and R image data may be displayed on a screen. The image output apparatus according to the present embodiment may include a parallax barrier capable of filtering the superimposed image being displayed. The image output apparatus according to the present embodiment may include a controller (not shown) configured to control the parallax barrier. Referring to FIG. 4, the L image data may be output to a user's left eye, and the R image data may be output to a user's right eye. The controller may move the parallax barrier from side to side based on an external input and vary image data cut off by the parallax barrier and image data provided to the user through slits. For example, the controller may control the parallax barrier to filter the superimposed image being displayed, so that only an L image or an R image can be output to both eyes of the user.

FIG. 5 illustrates examples of a first superimposed image including a plurality of image data being filtered by an image output apparatus 100, according to an embodiment of the present invention.

As shown in FIG. 5, the first superimposed image being filtered by the image output apparatus 100 may be an image obtained by indicating additional information regarding a target object on an image of the target object.
(a) of FIG. 5 shows a first superimposed image obtained by showing markers 515 indicating respective portions of the heart on an ultrasonic image 510 of the heart. In the image output apparatus 100 according to the present embodiment, the controller 120 may control the filtering unit 110 to filter the first superimposed image shown in (a) of FIG. 5 and output a second superimposed image including only the ultrasonic image 510 of the heart or only the markers 515.
(b) of FIG. 5 shows a first superimposed image obtained by showing a color Doppler image 525 on an ultrasonic image 520 of a parasternal long axis. In the image output apparatus 100 according to the present embodiment, the controller 120 may control the filtering unit 110 to filter the first superimposed image shown in (b) of FIG. 5 and output a second superimposed image including only the ultrasonic image 520 or only the color Doppler image 525.
(c) of FIG. 5 shows a first superimposed image obtained by showing a marker 535 indicating a direction in which imaginary light is irradiated, on a 3-dimensional ultrasonic image 530 of a fetus. In the image output apparatus 100 according to the present embodiment, the controller may control the filtering unit 110 to filter the first superimposed image shown in (c) of FIG. 5 and output a second superimposed image including only the 3-dimensional ultrasonic image 530 or only the marker 535.

In medical diagnostic images, a marker, an annotation, or color Doppler data may be displayed on an image of a target object and cover the image of the target object. In particular, when a color Doppler image occurs as a noise due to moving tissues, analyzing of the image of the target object may be hindered. Since a user of the image output apparatus 100 according to the present embodiment may control a ratio between a plurality of image data included in a superimposed image, the user may selectively view some of the image data. Accordingly, the user may remove various markers, annotations, or color Doppler information and make diagnoses while concentrating on an original scan image. Conversely, the user may selectively synthesize two images and utilize various pieces of marker information for diagnoses while minimizing a hindrance to analyzing images.

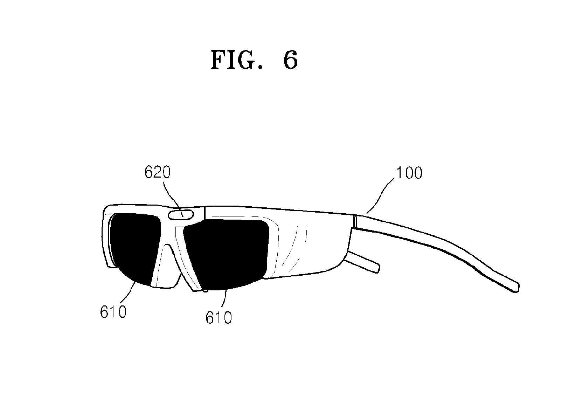

FIG. 6 is a diagram for explaining an image output apparatus 100 configured to receive an external input and control a filtering unit 110 using a wheel 620, according to an embodiment of the present invention.

Referring to FIG. 6, the image output apparatus 100 according to the present embodiment may be in the form of glasses. In this case, the filtering unit 110 may be included in lenses 610 included in glasses. Accordingly, a second superimposed image output by the filtering unit 110 may be an image recognized by a user through the lenses 610. In an example, as shown in FIG. 6, a controller 120 included in the image output apparatus 100 may include the wheel 620 configured to control the filtering unit 110 based on an external input. The user may control the wheel 620 and vary a ratio between image data output by the filtering unit 110.

An exemplary method of controlling the filtering unit 110 configured to filter a superimposed image including a first image and a second image using the wheel 620 will now be described. When the wheel 620 is disposed in the center of the image output apparatus 100, the filtering unit 110 may be controlled to output the first image to a user's left eye and output the second image to a user's right eye. When the wheel 620 is disposed at the end of a left side, the filtering unit 110 may be controlled to output an image including the first image and the second image at a ratio of about 1 : 0 to both eyes of the user.

The image output apparatus 100 according to the present embodiment may control a ratio between a plurality of image data included in a superimposed image so that the image data can be selectively provided to the user. Accordingly, a plurality of users may selectively view images of interest to each on only one screen. Also, a user may remove image data including noise or artifacts from the plurality of image data and view a superimposed image so that the remaining image data can be precisely determined without the influence of noise or artifacts.

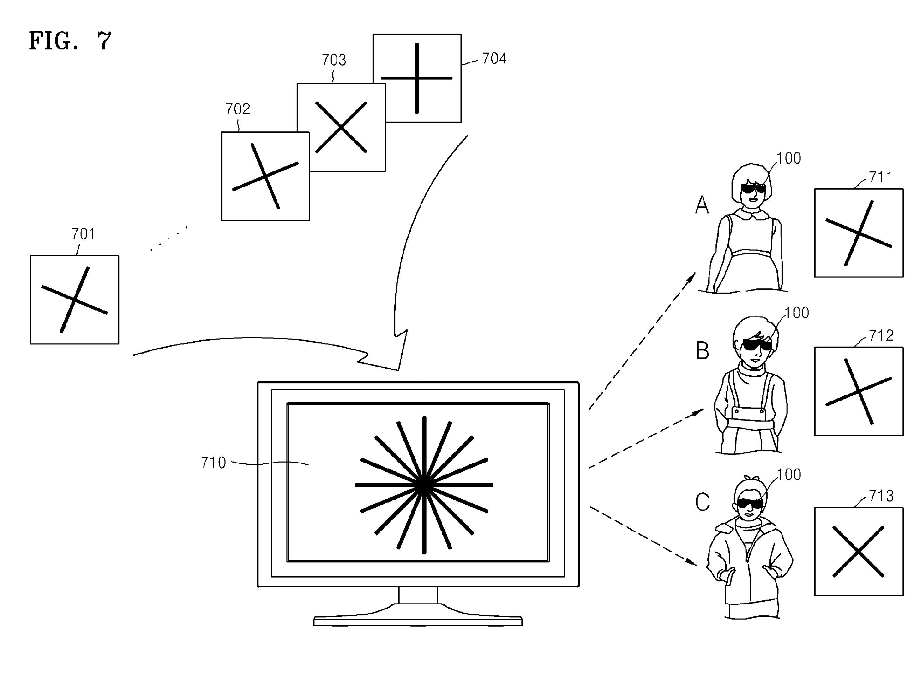

FIG. 7 is a diagram for explaining an image output apparatus configured to allow a plurality of users to receive different image data, according to an embodiment of the present invention.

Referring to FIG. 7, by using the image output apparatus 100, each of users A, B, and C may receive only interesting image data from one screen on which a superimposed image 710 including a plurality of image data 701, 702, 703, and 704 is displayed. The superimposed image 710 passing through the image output apparatus 100 may be recognized by the user A as an image 711. The superimposed image 710 passing through the image output apparatus 100 may be recognized by the user B as an image 712. The superimposed image 710 passing through the image output apparatus 100 may be recognized by the user C as an image 713.

For instance, when a user intends to display markers on a medical image of a target object or measure numerical values, for example, the length of a fetus' thighbone, a superimposed image including an image of the markers or the numerical values and an image of the target object may be provided to the user. In contrast, since the image of the markers or the numerical values is unnecessary for another user, a superimposed image excluding the image of the markers or the numerical values may be provided to the another user. According to the present invention, a plurality of users may selectively view images of interest from one screen without being affected by each other. Furthermore, when a user intends to use a superimposed image obtained by showing a color Doppler image on the image of the target object for diagnoses, the user may selectively receive only an image of some colors, out of the color Doppler image, using the image output apparatus 100 according to the present invention.

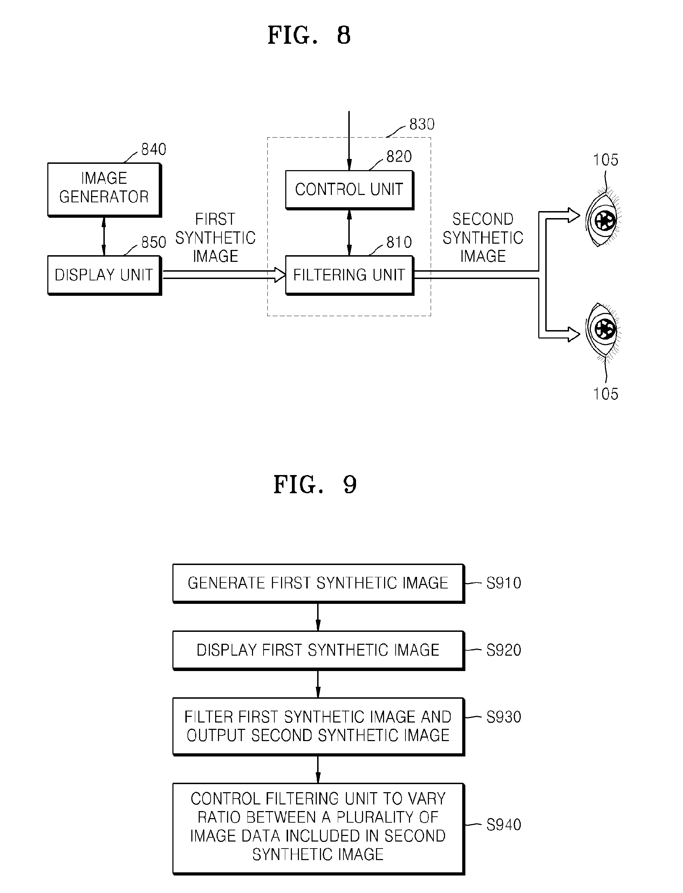

FIG. 8 is a block diagram of an image providing system 800 according to an embodiment of the present invention. The image providing system 800 may include an image generator 840, a display unit 850, and an image output unit 830. The image output unit 830 may include a filtering unit 810 and a controller 820.

The image generator 840 may generate a first superimposed image including a plurality of image data regarding a target object. The display unit 850 may display a first superimposed image generated by the image generator 840. The image output unit 830 may filter the first superimposed image displayed on the display unit 850 and output a second superimposed image. Since the filtering unit 810 and the controller 820 included in the image output unit 830 respectively correspond to the filtering unit 110 and the controller 120 of the image output apparatus 100 shown in FIG. 1, a description thereof will not be repeated here.

FIG. 9 is a flowchart illustrating a method of providing images according to an embodiment of the present invention. Referring to FIG. 9, the method of providing images according to the present embodiment may include operations being processed by the image providing system 800 shown in FIG. 8. Accordingly, even if omitted below, the above description of the image output apparatus 800 shown in FIG. 8 may be applied to the method of outputting images shown in FIG. 9.

In operation S91 0, the image providing system 800 may generate a first superimposed image including a plurality of image data regarding a target object. Operation S910 may be performed by the image generator 840.

In operation S920, the image providing system 800 may display the first superimposed image. Operation S920 may be performed by the display unit 850.

The image providing system 800 may generate and output a superimposed image including a plurality of image data using at least one of an anaglyph method, a polarization method, a shutter method, a parallax barrier method, and a lenticular lens method. In this case, since the superimposed image being generated and displayed by the image providing system 900 corresponds to a superimposed image being filtered by the image output apparatus 100, a description will not be repeated here.

In operations S930 and S940, the image providing system 800 may filter the first superimposed image and output a second superimposed image. Operation S930 may be performed by the filtering unit 810, and operation S940 may be performed by the controller 820. Since operations S930 and S940 correspond to operations S21 0 and S220 shown in FIG. 2, a description thereof will not be repeated here.

A method and apparatus for outputting images according to an embodiment of the present invention can control a ratio between a plurality of image data included in a superimposed image so that the image data can be selectively provided to the user. Accordingly, in the image output apparatus according to an embodiment of the present invention, a plurality of users can selectively view interesting images on only one screen without doing damage to each other. Also, in the image output apparatus according to an embodiment of the present invention, a user can remove image data including noise or artifacts from a plurality of image data and view a superimposed image so that the remaining image data may be precisely determined without the influence of the noise or artifacts.

The above-described various embodiments of the present invention may be embodied on a computer readable recording medium using, for example, software, hardware, or a combination thereof.

While this invention has been particularly shown and described with reference to exemplary embodiments thereof, it will be understood by those of ordinary skill in the art that various changes in form and details may be made therein without departing from the spirit and scope of the invention as defined by the appended claims. The exemplary embodiments should be considered in a descriptive sense only and not for purposes of limitation. Therefore, the scope of the invention is defined not by the detailed description of the invention but by the appended claims, and all differences within the scope will be construed as being included in the present invention.

## Claims

1. An image output apparatus comprising:
a filtering unit configured to filter a first superimposed image comprising a plurality of image data regarding a target object and to output a second superimposed image comprising the plurality of image data at a predetermined ratio; and
a controller configured to control the filtering unit to vary the ratio based on an external input.

2. The apparatus of claim 1, wherein the controller receives the external input using at least one of a wheel, a button, a wireless remote controller, a speech recognition sensor, and a gesture recognition sensor.

3. The apparatus of claim 1, further comprising a position information acquisition unit configured to obtain user position information,
wherein the controller controls the filtering unit based on the obtained user position information.

4. The apparatus of claim 1, wherein the controller controls the filtering unit such that the second superimposed image consists of at least one image data from among the plurality of image data.

5. The apparatus of claim 1, wherein the filtering unit filters the first superimposed image using at least one of an anaglyph method, a shutter method, a polarization method, a parallax barrier method, and a lenticular lens method.

6. The apparatus of claim 5, wherein the filtering unit using the anaglyph method filters the first superimposed image by controlling colors filtered through the filtering unit superimposed,
the filtering unit using the shutter method filters the first superimposed image by controlling opening and closing of a shutter included in the filtering unit superimposed,
the filtering unit using the polarization method filters the first superimposed image by controlling a polarization angle of a polarization filter included in the filtering unit superimposed,
the filtering unit using the parallax barrier method filters the first superimposed image by controlling a visual field cut off by a parallax barrier included in the filtering unit superimposed, and
the filtering unit using the lenticular lens method filters the first superimposed image by controlling an angle refracted by the lenticular lens included in the filtering unit and superimposed.

7. A method of outputting images, comprising:
filtering, by a filtering unit, a first superimposed image comprising a plurality of image data regarding a target object and outputting a second superimposed image comprising the plurality of image data at a predetermined ratio; and
controlling the filtering unit to vary the ratio based on an external output.

8. The method of claim 7, wherein the controlling of the filtering unit comprises receiving the external input using at least one of a wheel, a button, a wireless remote controller, a speech recognition sensor, and a gesture recognition sensor.

9. The method of claim 7, wherein the controlling of the filtering unit comprises:
obtaining user position information; and
controlling the filtering unit based on the obtained user position information.

10. The method of claim 7, wherein the outputting of the second superimposed image comprises filtering the first superimposed image using at least one of an anaglyph method, a shutter method, a polarization method, a parallax barrier method, and a lenticular lens method.

11. An image providing system comprising:
an image generator configured to generate a first superimposed image comprising a plurality of image data regarding a target object;
a display unit configured to display the first superimposed image; and
an image output unit comprising a filtering unit and a controller, the filtering unit being configured to filter the first superimposed image and to output a second superimposed image comprising the plurality of image data at a predetermined ratio, the controller being configured to control the filtering unit to vary the ratio.

12. The system of claim 11, wherein the controller controls the filtering unit to vary the ratio based on an external input.

13. The system of claim 11, wherein the image generator generates the first superimposed image comprising at least two image data obtained using different medical imaging methods.

14. The system of claim 11, wherein the image generator generates the first superimposed image comprising a first image and a second image, the first image showing the target object, the second image showing additional information regarding the target object.

15. A computer-readable medium having embodied thereon a computer program for executing the method of any one of claims 7-10.
